Europäisches Patentamt

European Patent Office

Office européen des brevets

(11) Veröffentlichungsnummer: **0 462 295 A1**

# (12) EUROPÄISCHE PATENTANMELDUNG

(21) Anmeldenummer: **90111219.3**

(22) Anmeldetag: **13.06.90**

(51) Int. Cl.5: **A61B 6/00, A61B 6/10**

(43) Veröffentlichungstag der Anmeldung:
**27.12.91 Patentblatt 91/52**

(84) Benannte Vertragsstaaten:
**DE FR GB NL**

(71) Anmelder: **SIEMENS AKTIENGESELLSCHAFT**

Wittelsbacherplatz 2
W-8000 München 2(DE)

(72) Erfinder: **Zerl, Wolfgang, Dipl.-Ing. (FH)**
**Schützengraben 38**
**W-8522 Herzogenaurach(DE)**

(54) Medizinisches Diagnose- oder Therapiegerät.

(57) Die Erfindung betrifft ein medizinisches Diagnose- oder Therapiegerät mit einem Geräteteil (2), welches relativ zu einer Fläche (23) motorisch verstellbar ist, wobei ein mit einer Steuereinrichtung verbundener, an dem Geräteteil (2) angebrachter Sensor (S3) vorgesehen ist, der mit der Steuereinrichtung derart zusammenwirkt, daß ein Verstellen des Geräteteiles (2) unterbunden ist, wenn der Sensor (S3) an der Fläche oder einem zwischen der Fläche und dem Geräteteil (2) befindlichen Objekt (23) anliegt. Dabei ist vorgesehen, daß mittels des Sensor (S3) ein von der Höhe einer auf ihn ausgeübten Druckkraft abhängiges Signal erzeugbar ist und daß die Steuereinrichtung das erzeugte Signal mit einem Schwellwert vergleicht, bei dessen Erreichen sie ein weiteres Verstellen des Geräteteiles (2) unterbindet.

FIG 2

EP 0 462 295 A1

Die Erfindung betrifft ein medizinisches Diagnose- oder Therapiegerät mit einem Geräteteil, welches relativ zu einer Fläche motorisch verstellbar ist, wobei ein mit einer Steuereinrichtung verbundener, an dem Geräteteil oder der Fläche angebrachter Sensor vorgesehen ist, der mit der Steuereinrichtung derart zusammenwirkt, daß ein Verstellen des Geräteteiles unterbunden ist, wenn der Sensor an der Fläche oder dem Geräteteil oder einem zwischen der Fläche und dem Geräteteil befindlichen Objekt anliegt. Dabei kann es sich bei der Fläche beispielsweise um eine Begrenzungswand, z.B. den Boden, desjenigen Raumes handeln, in dem das Gerät aufgestellt ist. Bei der Fläche kann es sich aber auch um die Fläche einer anderen Gerätekomponente des Gerätes selbst handeln, wobei die Fläche in diesem Fall stationär oder selbst beweglich sein kann, je nachdem, ob die Gerätekomponente ortsfest oder verstellbar ist.

Derartig ausgebildete Geräte bieten den Vorteil, daß die Gefahr von Sach- und Personenschäden stark verringert ist, da Kollisionen des verstellbaren Geräteteiles mit der Fläche bzw. mit zwischen der Fläche und dem Geräteteil befindlichen Objekten, es kann sich hierbei auch um Patienten oder Bedienpersonen handeln, vermieden werden, sofern ein zuverlässiger Sensor verwendet wird.

Als Sensoren wurden in der Vergangenheit beispielsweise Schaltmatten verwendet, die zwei elektrisch leitfähige Elemente enthalten, zwischen denen durch Krafteinwirkung im Kollisionsfall ein elektrischer Kurzschluß entsteht, den die Steuereinrichtung zu detektieren vermag. Derartige Schaltmatten haben sich als nachteilig erwiesen, weil sie zum einen infolge von Korrosionserscheinungen nicht ausreichend zuverlässig arbeiten und zum anderen insbesondere dann, wenn sie aus einer Vielzahl von matrixartig angeordneten einzelnen Schaltelementen aufgebaut werden, einen sehr hohen Fertigungsaufwand und hohe Kosten nach sich ziehen.

Außerdem ist es bekannt, als Sensoren aus Blech geformte und federnd aufgehängte Schaltringe zu verwenden, die, wenn eine ausreichend hohe Kraft auf sie einwirkt, mit der Steuereinrichtung verbundene Mikroschalter betätigen. Auch hier ist ein erheblicher konstruktiver und finanzieller Aufwand erforderlich. Außerdem ist für eine zuverlässige Funktion der Sensoren eine exakte Justage der Mikroschalter erforderlich, was einen hohen zeitlichen und damit finanziellen Aufwand bedeutet.

Nachteilig an beiden bekannten Sensoren ist außerdem, daß eine von der speziellen Konstruktion des jeweiligen Sensors abhängige Mindestkraft erforderlich ist, um ein von der Steuereinrichtung auswertbares Signal zu erhalten, der Sensor also verhältnismäßig spät anspricht. Da zum einen aufgrund der vorhandenen Signallaufzeiten eine gewisse Zeit vergeht, bis der das verstellbare Geräteteil antreibende Motor von seiner Stromversorgung getrennt wird, und zum anderen das Geräteteil auch dann nicht sofort zum Stillstand kommt, sondern infolge der beteiligten Massenträgheiten noch "nachläuft", besteht daher trotz erfolgter Abschaltung noch die Gefahr von Sach- und Personenschäden.

Es ist außerdem häufig erforderlich, das verstellbare Geräteteil, insbesondere wenn es sich dabei um ein an einen Patienten zu applizierendes Geräteteil handelt, motorisch derart zu verstellen, daß es mit einer definierten, dem jeweiligen Behandlungsfall angepaßten Kraft an dem Körper des Patienten anliegt. Dies ist mit den bekannten Sensoren praktisch unmöglich, da diese lediglich auf eine einen bestimmten Mindestwert überschreitende Krafteinwirkung reagieren.

Der Erfindung liegt die Aufgabe zugrunde, ein Gerät der eingangs genannten Art so auszubilden, daß zum einen unter allen Umständen eine so rechtzeitige Abschaltung der motorischen Verstellung des Geräteteiles gewährleistet ist, daß Schäden an Sachen, insbesondere dem Gerät selbst, und/oder an Personen vermieden sind, und daß zum anderen eine definierte Anpreßkraft zwischen dem Geräteteil und einem zwischen dem Geräteteil und der Fläche befindlichen Körper eines Patienten einstellbar ist.

Nach der Erfindung wird diese Aufgabe dadurch gelöst, daß mit Hilfe des Sensors ein von der Höhe einer auf ihn ausgeübten Druckkraft abhängiges Signal erzeugbar ist und daß die Steuereinrichtung das erzeugte Signal mit einem Schwellwert vergleicht, bei dessen Erreichen sie ein weiteres Verstellen des Geräteteiles unterbindet. Da das erzeugte Signal von der Höhe der Druckkraft abhängt, ist es durch geeignete Wahl des Schwellwertes also ohne weiteres möglich, zu gewährleisten, daß das Geräteteil mit einer definierten Kraft an dem Körper eines Patienten anliegt. Dabei muß die zwischen dem Geräteteil und dem Körper des Patienten wirksame Druckkraft nicht notwendigerweise mit der zwischen dem Sensor und dem Körper des Patienten wirkenden Druckkraft identisch sein. Ebenfalls durch Wahl eines geeigneten Schwellwertes kann erreicht werden, daß die Steuereinrichtung die motorische Verstellung des Geräteteiles so frühzeitig unterbindet, daß auch durch "Nachlaufen" des Geräteteiles keine Sach- oder Personenschäden entstehen können.

Eine weitere grundlegende Ausführung der Erfindung, die dazu dient, Kollisionen des verstellbaren Geräteteiles mit einem im Verstellweg des Geräteteiles befindlichen Objekt, z.B. dem Körper eines Lebewesens, einer Wand, einem weiteren Geräteteil usw., zu verhindern, ist im Anspruch 2 angegeben. Die Vorteile dieser Ausführungsform ergeben sich aus den Erläuterungen zu der zuerst beschriebenen Ausführung.

Eine besonders vorteilhafte Variante der Erfindung sieht vor, daß der Sensor wenigstens in Verstellrichtung des Geräteteiles elastisch nachgiebig ist. Bei geeigneter Anordnung des Sensors ist dann sichergestellt, daß ausgehend von der ersten Berührung des Sensors mit dem Geräteteil bzw. der Fläche oder mit einem zwischen beiden befindlichen Objekt noch ein gewisser Weg zu Verfügung steht, bevor das Geräteteil selbst mit der Fläche oder dem Objekt kollidiert. Außerdem ist die Verwendung eines elastisch nachgiebigen Sensors dann von Vorteil, wenn gemäß einer Variante der Erfindung die Steuereinrichtung das mit Hilfe des Sensors erzeugte Signal mit einem weiteren, einer geringeren Druckkraft entsprechenden Schwellwert vergleicht, bei dessen Erreichen die Steuereinrichtung eine Verlangsamung der Verstellbewegung des Geräteteiles bewirkt. Diese Maßnahme ist insbesondere deshalb von Bedeutung, weil das Maß, um das das Geräteteil nach Unterbinden der motorischen Verstellung "nachläuft" um so geringer ist, je geringer seine Geschwindigkeit bei Unterbindung der motorischen Verstellung ist. Durch die angegebene Maßnahme läßt sich also das "Nachlaufen" in ganz erheblichem Maße verringern.

Eine besonders vorteilhafte Ausführungsform der Erfindung sieht vor, daß mittels des Sensors ein Signal erzeugbar ist, welches von der Höhe einer auf den Sensor wirkenden Flächenpressung abhängt. Da die zulässige Höhe einer auf einen Patienten einwirkenden Druckkraft sowohl im Hinblick auf Beeinträchtigungen des Wohlbefindens des Patienten als auch auf die Gefahr von Schmerzempfindungen bzw. Verletzungen unter anderem von der Größe der Fläche abhängt, über die die Druckkraft auf den Patienten einwirkt, eine Druckkraft einer bestimmten Höhe wird einen Patienten bei punktueller Einwirkung eher als bei flächenhafter Einwirkung schädigen, ist es von besonderem Vorteil, wenn das Signal des Sensors von der jeweils vorhandenen Flächenpressung abhängt. Es besteht dann nämlich die Möglichkeit, den Patienten vor unzulässig hohen Flächenpressungen, die bereits bei sehr geringen wirksamen Kräften schmerzhaft oder schädlich sein können, zu schützen.

Da die Steuerung von Geräten der eingangs genannten Art gewöhnlich mittels einer elektrischen oder elektronischen Steuereinrichtung erfolgt, ist es zweckmäßig, wenn mit Hilfe des Sensors ein elektrisches Signal erzeugbar ist. Gemäß einer Variante der Erfindung ist daher vorgesehen, daß der Sensor ein elastisch nachgiebiges Widerstandsmaterial enthält, dessen elektrischer Widerstand sich in Abhängigkeit von einer auf ihn ausgeübten Druckkraft und/oder Flächenpressung ändert. In diesem Falle ist es durch die Messung des durch das Widerstandsmaterial fließenden Stromes sehr leicht möglich, ein der Höhe der auf den Sensor ausgeübten Druckkraft und/oder Flächenpressung entsprechendes elektrisches Signal zu bilden. Dabei ist es besonders vorteilhaft, wenn der Sensor nach einer Ausführungsform der Erfindung als elastisch nachgiebiges Widerstandsmaterial einen Schaumstoff enthält, in den Graphitteilchen eingelagert sind. Derartige Schaumstoffe sind unter der Bezeichnung "MOS-Gummi" im Handel erhältlich und dienen normalerweise dazu, bei der Lagerung von integrierten MOS-Schaltkreisen deren Anschlußpins aufzunehmen, wodurch diese elektrisch leitend miteinander verbunden sind. Auf diese Weise ist der Schaltkreis vor Schäden geschützt, die dadurch entstehen können, daß an einzelne Anschlußpins beispielsweise infolge von statischen Aufladungen sehr hohe Spannungen gelangen. Die Eigenschaft von MOS-Gummi, seinen elektrischen Widerstandswert in Abhängigkeit von einer auf ihn einwirkenden Druckkraft zu verändern, und die Verwendungsmöglichkeit von MOS-Gummi als Sensor-Material sind bereits in der Zeitschrift "Elektor", November 1978, Seiten 11-58 bis 11-60 beschrieben worden.

Um das Widerstandsmaterial ohne nachteilige Auswirkungen auf dessen elastische Nachgiebigkeit elektrisch kontaktieren zu können, ist es vorteilhaft, wenigstens diejenige Oberfläche des Sensors, auf die die Druckkraft und/oder Flächenpressung einwirkt mit einer elektrisch leitfähigen Schicht zu versehen. Als flexible elektrisch leitfähige Schicht eignet sich übrigens elektrisch leitfähiger Gummi. Zur Vermeidung von Kurzschlüssen ist es zweckmäßig, wenn wenigstens die flexible elektrisch leitfähige Schicht mit einer flexiblen Isolierschicht versehen ist, wobei infolge der flexiblen Eigenschaften der elektrisch leitfähigen Schicht und der Isolierschicht eine Beeinträchtigung der elastischen Nachgiebigkeit des Widerstandsmaterials vermieden ist.

Ein Ausführungsbeispiel der Erfindung ist in den beigefügten Zeichnungen dargestellt. Es zeigen:

Fig. 1    in perspektivischer Darstellung ein medizinisches Gerät zur Behandlung eines Lebewesens mit akustischen Stoßwellen,

Fig. 2    ein Detail des Gerätes gemäß Fig. 1 in teilweise geschnittener Darstellung,

Fig. 3 und 4    der Erläuterung der Funktion des erfindungsgemäßen Gerätes dienende Prinzipdarstellungen, und

Fig. 5    ein Blockschaltbild des Gerätes gemäß den Fig. 1 und 2.

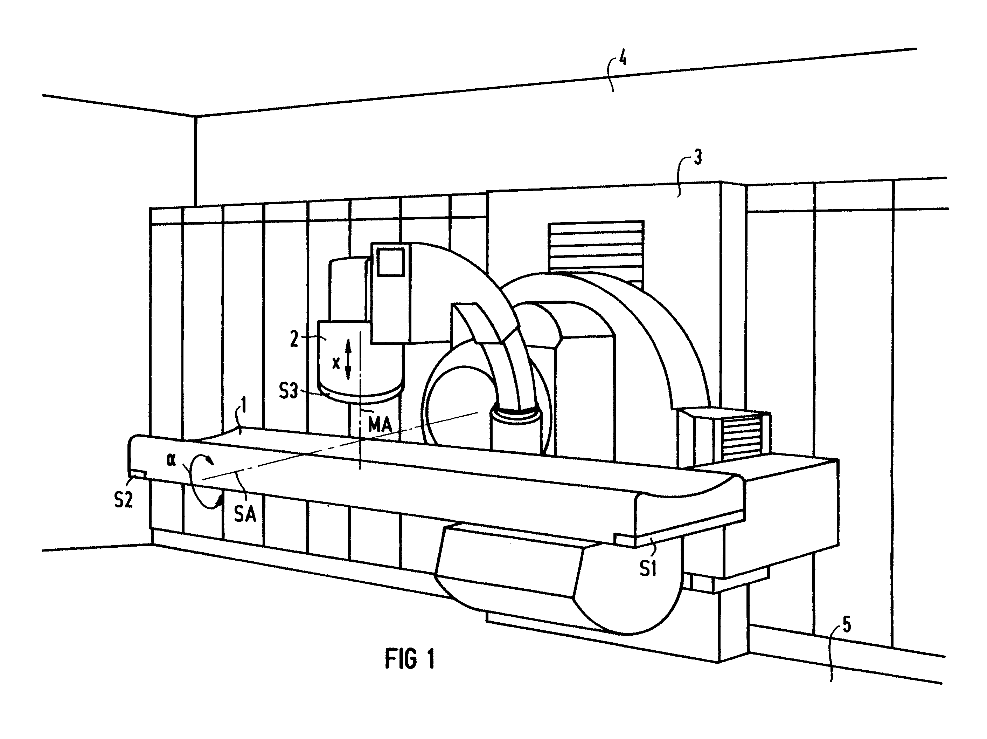

Das Gerät gemäß Fig. 1 weist als wesentliche Geräteteile einen Patientenlagerungstisch 1 und einen Stoßwellengenerator 2 auf, die beide an einem Ständer 3 angebracht sind, der an einer Wand 4 des Behandlungsraumes befestigt ist.

Um beispielsweise im Falle einer Lithotripsiebehandlung einen auf dem Patientenlagerungstisch 1 liegenden Patienten und den Stoßwellengenerator 2 relativ zueinander so positionieren zu können, daß sich der zu zertrümmernde Stein im Fokus der mittels des Stoßwellengenerators 2 erzeugbaren akustischen Stoßwellen befindet und der Stoßwellengenerator 2 zur akustischen Kopplung mit seinem Applikationsende an die Körperoberfläche des Patienten angepreßt ist, besteht unter anderem die Möglichkeit, den Patientenlagerungstisch 1 in Richtung des Doppelpfeiles "alpha" um eine Schwenkachse SA um ± 90° zu schwenken und den Stoßwellengenerator 2 in Richtung seiner Mittelachse MA in der durch den Doppelpfeil x angedeuteten Weise geradlinig zu verstellen. Die zu diesem Zweck in an sich bekannter Weise vorgesehenen Motore und Getriebe sind in Fig. 1 nicht dargestellt.

Um zu vermeiden, daß beim Schwenken des Patientenlagerungstisches 1 um die Schwenkachse SA das sich jeweils auf den Boden 5 des Behandlungsraumes zu bewegende Ende des Patientenlagerungstisches 1 mit dem Boden 5 kollidiert oder ein zwischen diesem Ende und dem Boden 5 befindliches Objekt, z.B. der Fuß einer Bedienperson, zwischen Patientenlagerungstisch 1 und Boden 5 eingequetscht wird, ist der Patientenlagerungstisch 1 an jedem seiner Enden an seiner Unterseite mit einem Sensor S1 bzw. S2 versehen. Außerdem ist an dem dem Patientenlagerungstisch 1 zugewandten Applikationsende des Stoßwellengenerators 2 ein weiterer Sensor S3 vorgesehen, der dazu dient, Kollisionen des Stoßwellengenerators 2 mit dem Patientenlagerungstisch bzw. ein Einquetschen eines zu behandelnden Patienten zwischen dem Stoßwellengenerator 2 und dem Patientenlagerungstisch 1 beim Schwenken des Patientenlagerungstisches 1 um die Schwenkachse SA und/oder beim Verstellen des Stoßwellengenerators 2 in der Richtung seiner Mittelachse MA zu verhindern.

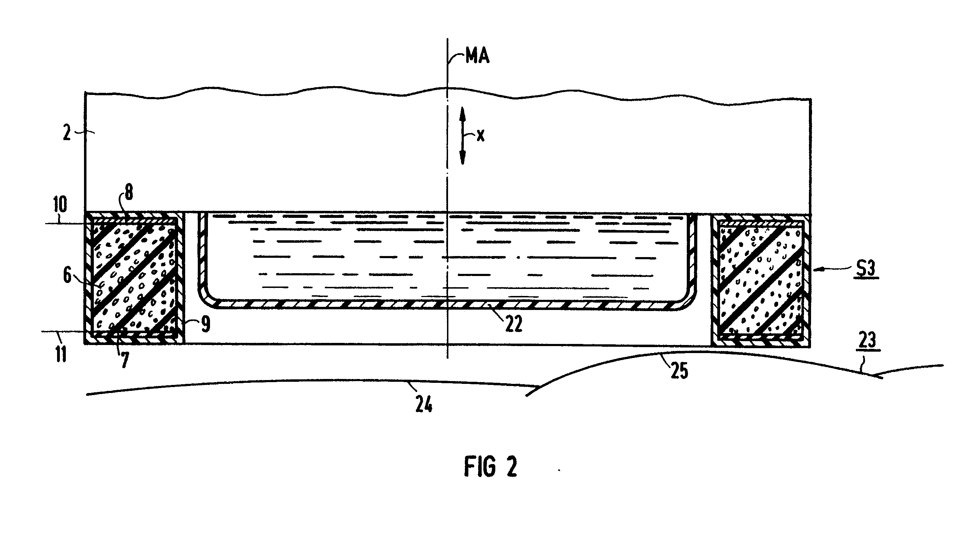

Der Aufbau der Sensoren S1 bis S3 ist in Fig. 2 am Beispiel des Sensors S3 verdeutlicht. Dieser ist von kreisringförmiger Gestalt und weist als wesentliches Bauelement einen Widerstandskörper 6 auf, der aus sogenanntem MOS-Gummi besteht. Wie schon erwähnt, handelt es sich hierbei um einen gummiartigen Schaumstoff, in den Graphitteilchen eingelagert sind. An seiner von dem Stoßwellengenerator 2 abgewandten Stirnfläche ist der Widerstandskörper 6 mit einer elektrisch leitfähigen, flexiblen Schicht 7 versehen, die die gesamte Stirnfläche des Widerstandskörpers 6 bedeckt. Die Schicht 7 besteht aus elektrisch leitfähigem Gummi. Die andere Stirnfläche des Widerstandskörpers 6 ist ebenfalls in ihrer Gesamtheit mit einer elektrisch leitfähigen Schicht 8 versehen, bei der es sich wie beispielsweise dargestellt um eine Metallfolie handeln kann. Bei Bedarf kann jedoch auch die Schicht 8 aus elektrisch leitfähigem Gummi bestehen. Die Schichten 7 und 8 sind mit dem Widerstandskörper 6 z.B. mittels eines geeigneten elektrisch leitenden Klebstoffes verbunden sind. Die durch den Widerstandskörper 6 und die Schichten 7 und 8 gebildete Anordnung ist insgesamt mit einer Isolierschicht 9 umhüllt, so daß außer über die durch die Isolierschicht 9 geführten und an den Schichten 7 bzw. 8 angebrachten Anschlußdrähte 10 und 11 keine elektrisch leitende Verbindung zu der innerhalb der Isolierschicht 9 befindlichen Anordnung hergestellt werden kann. Die Befestigung des Sensors S3 an dem Stoßwellengenerator 2 erfolgt durch Kleben. Die Sensoren S1 und S2 sind in zu Fig. 2 analoger Weise aufgebaut, mit dem Unterschied, daß diese Sensoren quaderförmig ausgebildet und mit dem Patientenlagerungstisch 1 verklebt sind.

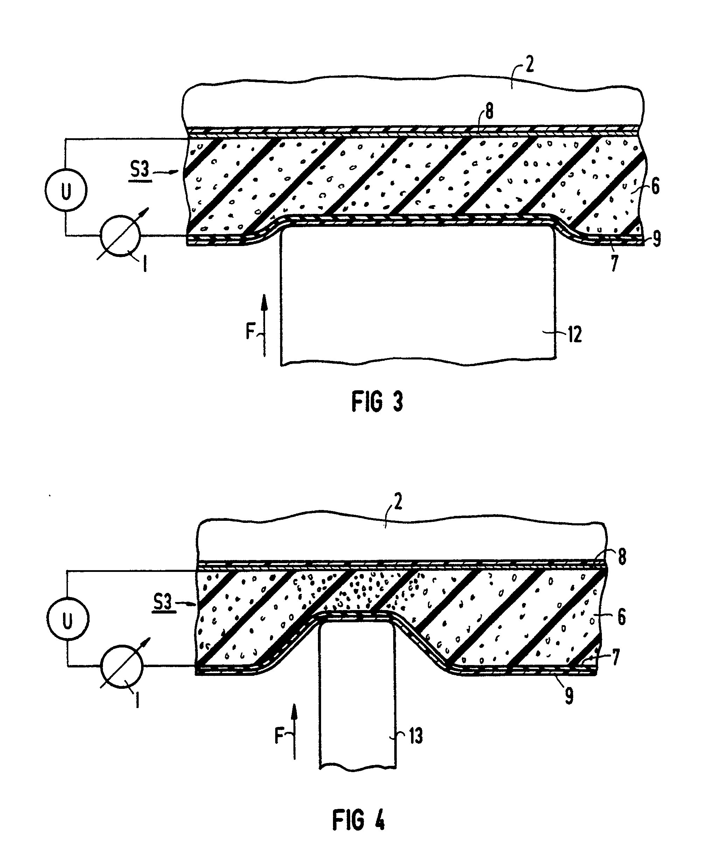

Die Wirkungsweise der Sensoren S1 bis S3 wird anhand der Fig. 3 und 4 deutlich, die schematisch den Sensor S3 im Schnitt in abgewickelter Darstellung zeigen.

Infolge des Umstandes, daß in dem den Widerstandskörper 6 bildenden MOS-Gummi in den Fig. 2 bis 4 als kleine Kreise schematisch angedeutete Graphitteilchen eingelagert sind, ist dieser wie erwähnt elektrisch leitfähig. Allerdings ist die Leitfähigkeit von MOS-Gummi nicht besonders gut, so daß ein daraus gebildeter Widerstandskörper in Abhängigkeit von der Größe des Widerstandskörpers und der Konzentration der Graphitteilchen in dem jeweils gewendeten MOS-Gummi einen Widerstandswert aufweist, der im Kiloohm- bis Megaohm-Bereich liegt.

Wirkt beispielsweise ein Fremdkörper 12 unter Ausübung einer Druckkraft F auf den Sensor S3 ein, wird der Widerstandskörper 6 im Bereich der Krafteinwirkung zusammengedrückt. Dadurch werden die in das Material des Widerstandskörper 6 eingelagerten Graphitteilchen ebenso zusammengepreßt, wodurch der elektrische Widerstand im Bereich der durch die Druckkraft F zusammengepreßten Stelle des Widerstandskörpers 6 sinkt. Wird über die Anschlußdrähte 10 und 11 eine konstante Spannung der Größe U an den Sensor S3 angelegt, nimmt infolge der Einwirkung der Druckkraft F der durch den Widerstandskörper 6 fließende Strom I gegenüber dem unbelasteten Zustand des Sensors S3 zu. Dabei hängt die Änderung des Stromes I von der im Bereich der Krafteinwirkung vorhandenen Flächenpressung ab. Dies verdeutlicht die Fig. 4, aus der ersichtlich ist, daß eine Druckkraft F der gleichen Größe wie im Falle der Fig. 4 eine stärkere Zusammenpressung des Widerstandskörpers 6 bewirkt, wenn sie mittels eines Fremdkörpers 13 auf den Sensor S3 ausgeübt wird, dessen Stirnfläche erheblich kleiner als die des Fremdkörpers 12 ist. Infolge dieser stärkeren Zusammenpressung des Widerstandskörpers 6 werden auch die in diesen eingelagerten Graphitteilchen im Bereich der Krafteinwirkung stärker zusammengepreßt, so daß im Bereich der Krafteinwirkung ein im Vergleich zur Fig. 3 geringerer Widerstandswert des Widerstandskörpers 6 vorliegt, obwohl eine Kraft der gleichen Größe wie im Falle der Fig. 3 auf ihn einwirkt. Im Falle der Fig. 4 wird also ein höherer Strom als im Falle der Fig. 3 fließen. Es versteht sich, daß die Höhe des Stromes für eine gegebene Größe der Angriffsfläche der Druckkraft mit größer werdender Druckkraft ebenfalls steigt. Die vorstehenden Ausführungen gelten übrigens für beliebig geformte Sensoren, also auch die Sensoren S1 und S2, sinngemäß.

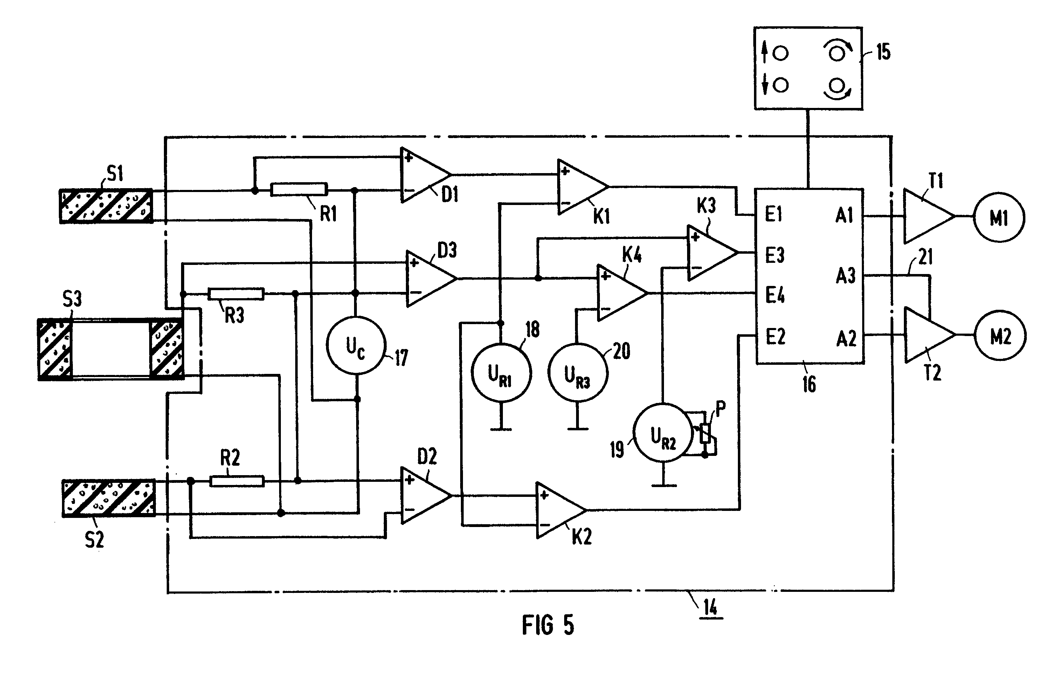

Die erläuterte Eigenschaft der Sensoren S1 bis S3, ihren Widerstandswert in Abhängigkeit von einer auf sie einwirkenden Druckkraft bzw. Flächenpressung zu ändern, wird im Falle des erfindungsgemäßen Gerätes wie bereits angedeutet dazu genutzt, Kollisionen des Patientenlagerungstisches 1 mit dem Boden 5 sowie des Stoßwellengenerators 2 mit dem Patientenlagerungstisch 1 zu unterbinden. Außerdem wird das beschriebene Verhalten der Sensoren S1 bis S3 dazu ausgenutzt, zu verhindern, daß ein Patient zwischen dem Stoßwellengenerator 2 und dem Patientenlagerungstisch 1 bzw. Körperteile einer Bedienperson zwischen dem Patientenlagerungstisch 1 und dem Boden 5 eingequetscht werden können. Zu diesem Zweck sind die Sensoren S1 bis S3 gemäß Fig. 5 an eine noch im einzelnen zu beschreibende Steuereinheit 14 angeschlossen, welche dazu dient, Elektromotore M1 und M2 über Treiberstufen T1 und T2 in Abhängigkeit davon anzusteuern, in welcher Weise eine an die Steuereinheit 14 angeschlossene Bedieneinheit 15 betätigt wird. Dabei dient der Motor M1 dazu, die Patientenliege 1 in der einen oder anderen Richtung um die Schwenkachse SA zu schwenken. Die Bedieneinheit 15 weist ein durch entsprechende gekrümmte Pfeile gekennzeichnetes Tastenpaar auf, wobei die Steuereinheit 14 bei Betätigung einer der Tasten des genannten Tastenpaares den Motor M1 über die Treiberstufe T1 in der entsprechenden Richtung so lange ansteuert, wie die Taste gedrückt ist. Der Motor M2 dient dazu, den Stoßwellengenerator 2 in Richtung seiner Mittelachse MA zu verstellen, wenn eine der Tasten eines mit entsprechenden Pfeilen bezeichneten zweiten Tastenpaares der Bedieneinheit 15 betätigt wird. Dabei steuert auch hier die Steuereinheit 14 den Motor M2 über die Treiberstufe T2 in der der gedrückten Taste des genannten Tastenpaares entsprechenden Richtung so lange an, wie diese Taste gedrückt ist.

Die Ansteuerung der Motore M1 und M2 nimmt die Steuereinheit 14 in der beschriebenen Weise nur so lange vor, solange an keinem der Eingänge E1 bis E3 einer in der Steuereinheit 14 enthaltenen Steuerlogik 16, mit deren Ausgängen A1 und A2 die Treiberstufen T1 und T2 verbunden sind, ein Signal anliegt, das darauf hindeutet, daß eine Kollision eines der Enden des Patientenlagerungstisches 1 mit dem Boden 5 vorliegt bzw. ein Objekt zwischen einem der Enden des Patientenlagerungstisches 1 und dem Boden 5 eingeklemmt ist oder daß eine Kollision des Stoßwellengenerators 2 mit dem Patientenlagerungstisch 1 vorliegt bzw. der Stoßwellengenerator 2 mit einer unzulässig hohen Kraft gegen einen auf dem Patientenlagerungstisch 1 liegenden Patienten 1 angepreßt ist.

Um diese Signale erzeugen zu können, sind die Sensoren S1, S2, S3 in Parallelschaltung mit einer Konstantspannungsquelle 17 verbunden, wobei jeweils in eine der beiden einen Sensor S1, S2, S3 mit der Konstantspannungsquelle 17 verbindenden Leitungen ein Shuntwiderstand R1, R2, R3 geschaltet ist. Die ein Maß für die durch die Sensoren S1, S2, S3 fließenden Ströme darstellenden Spannungsabfälle über den Shuntwiderständen R1, R2, R3 werden mit Hilfe von schematisch angedeuteten Differenzverstärkern D1, D2, D3 gemessen. Die Ausgangssignale der Differenzverstärker D1, D2, D3 sind jeweils dem positiven Eingang von schematisch angedeuteten Komparatoren K1, K2, K3 zugeführt. Den negativen Eingängen der Komparatoren K1 und K2 ist jeweils die mittels einer Referenzspannungsquelle erzeugte Referenzspannung $U_{R1}$ zugeführt. Dem negativen Eingang des Komparators K3 ist eine mittels einer Referenzspannungsquelle 19 erzeugte Referenzspannung $U_{R2}$ zugeführt, wobei die Möglichkeit besteht, die Höhe der Referenzspannung $U_{R2}$ zu verändern, was durch ein mit der Referenzspannungsquelle 19 verbundenes Potentiometer P angedeutet ist. Solange die Ausgangssignale der Differenzverstärker D1, D2, D3 geringer als die den negativen Eingängen der Komparatoren K1, K2, K3 zugeführten Referenzspannungen $U_{R1}$ bzw. $U_{R2}$ sind, liegen die Pegel an den Ausgängen der Komparatoren K1, K2, K3 auf ihren unteren Extremwerten, beispielsweise logisch "0". Solange diese Pegel an den Eingängen E1, E2 und E3 der Steuerlogik 16 liegen, steuert die Steuerlogik 16 die Motore M1 und M2 in der Weise an, wie dies eine eventuelle Betätigung der Tasten der Bedieneinheit 15 erfordert. Liegt jedoch an einem der Eingänge E1, E2, E3 der Steuerlogik 16 ein Signal, das dem oberen Extremwert des Ausgangspegels, z.B. logisch "1", des entsprechenden Komparators K1, K2, K3 entspricht, unterbindet die Steuerlogik 16 die Ansteuerung der Motore M1 und M2 im Sinne bestimmter Gerätebewegungen.

Im einzelnen unterbindet die Steuerlogik 16 ein Schwenken des Patientenlagerungstisches 1 im Uhrzeigersinn, wenn an ihrem Eingang E1 ein Signal des Pegels logisch "1" anliegt. Umgekehrt unterbindet die Steuerlogik 16 ein Schwenken des Patientenlagerungstisches gegen den Uhrzeigersinn, wenn an ihrem Eingang E2 ein Signal des Pegels logisch "1" anliegt. Ein Verstellen des Stoßwellengenerators 2 in Richtung auf den Patientenlagerungstisch 1 sowie Schwenkbewegungen des Patientenlagerungstisches 1 sowohl im Uhrzeigersinn als auch gegen den Uhrzeigersinn unterbindet die Steuerlogik 16, wenn an ihrem Eingang E3 ein Signal des Pegels logisch "1" anliegt. Es wird also deutlich, daß ein Schwenken des Patientenlagerungstisches 1 in der einen oder anderen Richtung unterbunden ist, wenn der Sensor S1 bzw. S2 durch Kontakt mit dem Boden 5 bzw. einem auf dem Boden 5 befindlichen Fremdkörper, z.B. dem Fuß einer Bedienperson, so weit verformt wird, däß das der hierdurch bewirkten Widerstandsänderung des Sensors S1 bzw. S2 entsprechende Ausgangssignal des Differenzverstärkers D1 bzw. D2 die Referenzspannung $U_{R1}$ überschreitet und so ein Umspringen des Ausgangspegels des Komparators K1 bzw. K2 bewirkt. In entsprechender Weise wird ein Verstellen des Stoßwellengenerators 2 in Richtung auf den Patientenlagerungstisch 1 und ein Schwenken des Patientenlagerungstisches 1 in beiden Richtungen unterbunden, wenn der Sensor S3 durch Kontakt mit dem Patientenlagerungstisch 1 oder einem zwischen dem Stoßwellengenerator 2 und dem Patientenlagerungstisch befindlichen Objekt, z.B. dem Körper eines Patienten, so weit zusammengepreßt wird, daß das der hierdurch bewirkten Widerstandsänderung entsprechende Ausgangssignal des Differenzverstärkers D3 die mittels des Potentiometers D eingestellte Referenzspannung $U_{R2}$ übersteigt und somit den Ausgangspegel des Komparators K3 umspringen läßt.

Die Referenzspannung $U_{R1}$ ist derart gewählt, daß auch unter Berücksichtigung des "Nachlaufens" des Patientenlagerungstisches 1 weder Schäden an dem Patientenlagerungstisch 1 selbst oder an zwischen diesem und dem Boden 5 befindlichen Gegenständen, sofern es sich hierbei um in dem Behandlungsraum üblicherweise vorhandene, nicht allzu zerbrechliche Gegenstände handelt, noch an zwischen dem Patientenlagerungstisch 1 und dem Boden 5 befindlichen Körperteilen von in dem Behandlungsraum anwesenden Personen entstehen können. Die Referenzspannung $U_{R2}$ wird mittels des Potentiometers P dem jeweiligen Behandlungsfall entsprechend so eingestellt, daß Verletzungen des Patienten völlig ausgeschlossen sind und einem Patienten unter normalen Umständen keine Schmerzen zugefügt werden können, wenn der Stoßwellengenerator 2 mit der Körperoberfläche des Patienten in Eingriff gebracht wird.

Die Steuereinheit 14 enthält außerdem einen Komparator K4, dessen positivem Eingang das Ausgangssignal des Differenzverstärkers D3 zugeführt ist. Dem negativen Eingang des Komparators K4 ist die mittels einer weiteren Referenzspannungsquelle 20 erzeugte Referenzspannung $U_{R3}$ zugeführt. Diese ist größer als der maximal einstellbare Wert der Referenzspannung $U_{R2}$. Der Ausgang des Komparators K4 ist mit dem Eingang E4 der Steuerlogik 16 verbunden. Die Steuerlogik 16 weist außerdem einen Ausgang A3 auf, an den eine Steuerleitung 21 angeschlossen ist, die zu der Treiberstufe T2 führt. In Abhängigkeit davon, ob der Ausgang A3 den Pegel logisch "0" oder den Pegel logisch "1" führt, steuert die Treiberstufe T2 den Motor M2 derart an, daß dieser den Stoßwellengenerator 2 entweder mit normaler oder reduzierter Geschwindigkeit verstellt. Es wird also deutlich, daß, solange der Pegel des Ausgangssignales des Differenzverstärkers D3 die Referenzspannung $U_{R3}$ nicht überschreitet, die Steuerlogik 16 die Treiberstufe T2 bei entsprechender Betätigung der Bedieneinheit 15 derart ansteuert, daß der Motor M2 den Stoßwellengenerator 2 mit normaler Geschwindigkeit verstellt. Sobald der an dem Stoßwellengenerator 2 angebrachte Sensor S3 durch Kollision mit dem Patientenlagerungstisch 1 oder einem auf diesem liegenden Objekt, z.B. einem Patienten, so weit verformt wird, daß infolge der dabei auftretenden Widerstandsänderung das Ausgangssignal des Differenzverstärkers D3 die Referenzspannung $U_{R3}$ übersteigt, steuert die Steuerlogik 16 die Treiberstufe T2 derart an, daß der Motor M2 den Stoßwellengenerator 2 mit reduzierter Geschwindigkeit verstellt. Infolge der reduzierten Geschwindigkeit ist gewährleistet, daß ein "Nachlaufen" des Stoßwellengenerators 2 praktisch nicht auftritt, wenn die Steuerlogik 16 wegen eines die Referenzspannung $U_{R2}$ übersteigenden Pegels des Ausgangssignals des Differenzverstärkers D3 die motorische Verstellung des Stoßwellengenerators 2 in Richtung auf die Patientenliege 1 unterbindet.

Bekanntermaßen besteht die Möglichkeit, einen Stoßwellengenerator in der Weise an den Körper eines zu behandelnden Patienten anzukoppeln, daß er mittels eines einen ein akustisches Ausbreitungsmedium für die Stoßwellen enthaltenden Raum abschließenden flexiblen Sackes an die Körperoberfläche des Patienten angepreßt wird. Einen derartigen mit 22 bezeichneten flexiblen Sack weist auch der Stoßwellengenerator 2 auf (siehe Fig. 2). Der flexible Sack 22 ist von dem Sensor S3 umgeben. Dabei besitzt der Sensor S3 in Richtung der Mittelachse MA des Stoßwellengenerators 2 gemessen eine solche Höhe, daß er den flexiblen Sack 22 überragt. Demzufolge kommt die von dem Stoßwellengenerator 2 abgewandte Stirnfläche des Sensors S3 beim Verstellen des Stoßwellengenerators 2 in Richtung auf den Patientenlagerungstisch 1 mit der Oberfläche eines in Fig. 2 schematisch angedeuteten Körpers 23 eines Patienten in Eingriff, bevor der Sack 22 an der Oberfläche des Körpers 23 anliegt. Die Höhe der Referenzspannung $U_{R2}$ wird dem jeweiligen Behandlungsfall entsprechend so eingestellt, daß das Ausgangssignal des Differenzverstärkers D3 die Referenzspannung $U_{R2}$ dann überschreitet, wenn der Sack 22 mit leichtem Druck flächenhaft und gleichmäßig an der Oberfläche des Körpers 23 anliegt. Dies ist z.B. dann der Fall, wenn der Stoßwellengenerator 2 mit seinem Sack 22 an der mit 25 bezeichneten Bauchdecke anliegt. Ist der Stoßwellengenerator 2 relativ zu dem Körper 23 des zu behandelnden Patienten jedoch wie in Fig. 2 dargestellt so ausgerichtet, daß der Sensor S3 und der Sack 22 zunächst im Bereich des mit 25 bezeichneten Rippenbogens mit dem Körper 23 in Eingriff kommen, werden Sensor S3 und Sack 22 im Bereich des Rippenbogens 25 sehr viel stärker verformt als dies bei einer gleichmäßigen Anlage des Sackes 22 beispielsweise im Bereich der Bauchdecke 24 der Fall ist. Demnach wird lokal eine sehr hohe Flächenpressung auf den Sensor S3 ausgeübt mit der Folge, daß die Steuerlogik 16 eine weitere motorische Verstellung des Stoßwellengenerators 2 in Richtung auf den Patientenlagerungstisch 1 bzw. den Körper 23 des Patienten unterbindet, bevor der Sack 22 vollständig an der Oberfläche des Körpers 23 anliegt. Dies ist durchaus wünschenswert, da eine Druckkraft, die von dem Patienten ohne weiteres ertragen werden kann, wenn sie über die Fläche des Sackes 22 verteilt auf die Bauchdecke 24 einwirkt, Schmerzen oder sogar Verletzungen verursachen kann, wenn sie im Bereich des Rippenbogens 25 lokal konzentriert auf den Körper 23 des Patienten einwirkt.

Die Erfindung wird ausschließlich am Beispiel eines medizinischen Gerätes zur Behandlung eines Lebewesens mit akustischen Stoßwellen beschrieben. Sie kann aber auch bei anderen medizinischen Diagnose- oder Therapiegeräten, z.B. Röntgendiagnostikgeräten, strahlentherapeutischen Geräten usw., Verwendung finden.

## Patentansprüche

1. Medizinisches Diagnose- oder Therapiegerät mit einem Geräteteil (1, 2), welches relativ zu einer Fläche (5, 2 bzw. 1) motorisch verstellbar ist, wobei ein mit einer Steuereinrichtung (14) verbundener, an dem Geräteteil (1, 2) oder der Fläche (2, 1) angebrachter Sensor (S1, S2, S3) vorgesehen ist, der mit der Steuereinrichtung (14) derart zusammenwirkt, daß ein Verstellen des Geräteteiles (1, 2) unterbunden ist, wenn der Sensor (S1, S2 bzw. S3) an der Fläche (5 bzw. 1) oder dem Geräteteil (1) oder einem zwischen der Fläche (1 bzw. 5) und dem Geräteteil (2 bzw. 1) befindlichen Objekt anliegt, **dadurch gekennzeichnet,** daß mit Hilfe des Sensors (S1, S2, S3) ein von der Höhe einer auf ihn ausgeübten Druckkraft abhängiges Signal erzeugbar ist und daß die Steuereinrichtung (14) das erzeugte Signal mit einem Schwellwert ($U_{R1}$, $U_{R2}$) vergleicht, bei dessen Erreichen sie ein weiteres Verstellen des Geräteteiles (1, 2) unterbindet.

2. Medizinisches Diagnose- oder Therapiegerät mit einem Geräteteil (1, 2), welches motorisch verstellbar ist, wobei ein mit einer Steuereinrichtung (14) verbundener, an dem Geräteteil (1, 2) angebrachter Sensor (S1, S2, S3) vorgesehen ist, der mit der Steuereinrichtung (14) derart zusammenwirkt, daß ein Verstellen des Geräteteiles (1, 2) unterbunden ist, wenn der Sensor (S1, S2 bzw. S3) an einem im Verstellweg des Geräteteiles (1, 2) befindlichen Objekt (5 bzw. 1, 23) anliegt, **dadurch gekennzeichnet,** daß mit Hilfe des Sensors (S1, S2, S3) ein von der Höhe einer auf ihn ausgeübten Druckkraft abhängiges Signal erzeugbar ist und daß die Steuereinrichtung (14) das erzeugte Signal mit einem Schwellwert ($U_{R1}$, $U_{R2}$) vergleicht, bei dessen Erreichen sie ein weiteres Verstellen des Geräteteiles (1, 2) unterbindet.

3. Gerät nach Anspruch 1 oder 2, **dadurch gekennzeichnet,** daß der Sensor (S1, S2, S3) wenigstens in Verstellrichtung des Geräteteiles (1, 2) elastisch nachgiebig ist.

4. Gerät nach Anspruch 3, **dadurch gekennzeichnet,** daß die Steuereinrichtung (14) das mit Hilfe des Sensors (S3) erzeugte Signal mit einem weiteren, einer geringeren Druckkraft entsprechenden Schwellwert ($U_{R3}$) vergleicht, bei dessen Erreichen die Steuereinrichtung (14) eine Verlangsamung der Verstellbewegung des Geräteteiles (2) bewirkt.

5. Gerät nach einem der Ansprüche 1 bis 4, **dadurch gekennzeichnet,** daß mittels des Sensors (S1, S2, S3) ein Signal erzeugbar ist, welches von der Höhe einer auf den Sensor (S1, S2, S3) wirkenden Flächenpressung abhängt.

6. Gerät nach einem der Ansprüche 1 bis 5, **dadurch gekennzeichnet,** daß mit Hilfe des Sensors (S1, S2, S3) ein elektrisches Signal erzeugbar ist.

7. Gerät nach Anspruch 6, **dadurch gekennzeichnet**, daß der Sensor (S1, S2, S3) ein elastisch nachgiebiges Widerstandsmaterial enthält, dessen elektrischer Widerstand sich in Abhängigkeit von einer auf ihn ausgeübten Druckkraft und/oder Flächenpressung ändert.

8. Gerät nach Anspruch 7, **dadurch gekennzeichnet**, daß der Sensor (S1, S2, S3) als elastisch nachgiebiges Widerstandsmaterial (6) einen Schaumstoff enthält, in dem Graphitteilchen eingelagert sind.

9. Gerät nach Anspruch 8, **dadurch gekenn-zeichnet**, daß wenigstens diejenige Oberflä-che des Sensors (S1, S2, S3), auf die die Druckkraft und/oder die Flächenpressung einwirkt, mit einer flexiblen elektrisch leitfähigen Schicht (7) versehen ist.

10. Gerät nach Anspruch 9, **dadurch gekennzeichnet**, daß wenigstens die flexible elektrisch leitfähige Schicht (7) mit einer flexiblen Isolierschicht (9) versehen ist.

FIG 1

EP 0 462 295 A1

FIG 2

EP 0 462 295 A1

FIG 3

FIG 4

FIG 5

EP 0 462 295 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | GB-A-1 098 547 (GENERAL ELECTRIC CO., LTD) * Seite 2, Zeilen 26-54; Seite 2, Zeile 86 - Seite 3, Zeile 60; Figuren 1-3 * | 1-3,5,6 | A 61 B 6/00 A 61 B 6/10 |
| X | DE-A-2 356 276 (VEB TRANSFORMATOREN- UND RÖNTGENWERK HERMANN MATERN) * Seite 3, Zeile 16 - Seite 5, Zeile 34; Seite 7, Zeile 13 - Seite 8, Zeile 24; Seite 9, Zeile 24 - Seite 10, Zeile 14; Figuren 1-3 * | 1-3,5 | |
| A | | 4 | |
| X | EP-A-0 301 198 (DORNIER MEDIZINTECHNIK GmbH) * Spalte 1, Zeile 46 - Spalte 3, Zeile 3; Figuren 1,2 * | 1-3,5 | |
| X | DE-A-2 158 457 (BROWN BOVERI & CIE AG) * Seite 3, Zeile 11 - Seite 4, Zeile 23; Seite 7, Zeilen 13-17; Seite 9, Zeilen 5-23; Seite 11, Zeilen 18-22; Figuren 1-4 * | 1,2,5-7 | |
| A | | 3 | |
| A | DE-A-2 739 934 (SIEMENS AG) * Seite 2, Zeile 18 - Seite 4, Zeile 31; Seite 5, Zeile 28 - Seite 6, Zeile 26; Figuren 1-3 * | 1-3,5-10 | |
| A | FR-A-2 273 257 (AGENCE NATIONALE DE VALORISATION DE LA RECHERCHE) * Seite 2, Zeile 17 - Seite 3, Zeile 24; Seite 4, Zeilen 4-12; Figuren 1-12 * | 5-10 | |

**RECHERCHIERTE SACHGEBIETE (Int. Cl.5)**

A 61 B

Der vorliegende Recherchenbericht wurde für alle Patentansprüche erstellt

| Recherchenort | Abschlußdatum der Recherche | Prüfer |
|---|---|---|
| Den Haag | 04 Februar 91 | FONTENAY P.H.E.V. |